# EUROPEAN PATENT SPECIFICATION

(11) **EP 3 055 012 B1**
(45) Date of publication and mention of the grant of the patent: **26.09.2018**
(21) Application number: 14851799.8
(22) Date of filing: 06.10.2014
(51) Int. Cl.: A61M 16/04, A61B 1/267

(54) **SYSTEMS FOR SELECTIVELY BLOCKING RESPIRATORY AIR FLOW**
SYSTEME ZUR SELEKTIVEN BLOCKIERUNG DES BEATMUNGSLUFTSTROMS
SYSTÈMES POUR BLOQUER SÉLECTIVEMENT UN FLUX D'AIR RESPIRATOIRE

(30) Priority: 07.10.2013 US 201361887814 P
(43) Date of publication of application: 17.08.2016
(73) Proprietor: Endoclear LLC, San Ramon, CA 94583 (US)
(72) Inventor: VAZALES, Brad Eugene, San Ramon, CA 94583 (US)
(74) Representative: Rees, Kerry
(86) International application number: PCT/US2014/059247
(87) International publication number: WO 2015/054102

(56) References cited:
- WO-A1-2011/097439
- WO-A1-2011/126812
- US-A1- 2006 090 761
- US-A1- 2006 135 947
- US-A1- 2013 104 884

## Description

### Field

The embodiments described herein generally relate to the ability to selectively ventilate one lung or portion thereof during ventilation of a patient through an endotracheal tube or other body-inserted medical tube.

### Background

Ventilation of a patient through a single-lumen endotracheal tube results in essentially equal ventilation of both lung fields simultaneously. There are instances when it may be clinically advantageous to be able to ventilate only one lung at a given time. For example, the most common clinical situation when ventilation of only one lung is desired is during thoracic surgery (either open or thoracoscopic), in which, for visualization and technical reasons, it may be necessary to cease ventilation to the lung that is being operated upon. A less common clinical situation can occur when there is pathology in one lung (such as excessive air leak, hemorrhage, or infection) that needs to be mechanically isolated from the normally-functioning lung.

There are currently two main options for single-lung isolation during ventilation. The first option is the use of a double-lumen endotracheal tube, where one lumen of the endotracheal tube is placed into either the right or left main stem bronchus and a balloon is inflated within that bronchus. Subsequently, at the discretion of the clinician, ventilation can be accomplished utilizing only the lung whose main stem was intubated, using only the other lung whose main stem was not intubated, or through a Y-connector that allows ventilation of both lungs. Double-lumen endotracheal tubes can be somewhat stiff and difficult to position. Appropriate positioning can be assessed either through indirect clinical measurements, such as breath sounds, or utilizing a pediatric-size bronchoscope to visually verify its correct placement. The stiffness and size of these double-lumen endotracheal tubes has been associated with bronchial mucosal injury, such as hemorrhage. Once the double-lumen tube is confirmed in position and the surgical procedure is underway, possible dislodgement or other difficulties with ventilation require reinserting a bronchoscope, often with the patient not in a favorable anatomic position, to try to reposition the double-lumen tube.

The second option for single-lung isolation during ventilation is to use a bronchial blocker. In its simplest form, the bronchial blocker is a balloon catheter that is placed through a single-lumen endotracheal tube and then into either the right or left main stem bronchus. Once the balloon is inflated, ventilation only occurs in the opposite lung. Usually, the bronchial blocker is placed utilizing a bronchoscope which, since the bronchoscope and bronchial blocker are both within the endotracheal tube, can cause temporary airway obstruction or difficulty in ventilating the patient.
WO 2011/126812 discloses systems, methods, and devices for facilitating insertion of an endotracheal tube and/or for verifying the position of the endotracheal tube within an airway of a patient with respect to an anatomical landmark of a patient. The document also discloses systems, methods, and devices for facilitating removal of debris from the distal airways of a patient under direct visualization.
WO 2011/097439 discloses a single lumen endobronchial tube including a medical tube having a single lumen with an opening at each of opposed distal and proximal ends of the tube, the opening at the proximal end of the tube being adapted for connection to an external mechanical ventilation device, and the opening at the distal end of the tube being adapted for delivery of a medical gas; a wall extending throughout the tube's entire length having an internal wall surface, an external wall surface and a thickness therebetween, a portion of the wall having an aperture and a shaft adapted to house a mechanism for sealing the aperture; a distal bronchial cuff positioned along the external wall surface and adapted to expand radially outward; and at least a first proximal tracheal cuff positioned along the external wall surface and adapted to expand radially outward.

### SUMMARY

According to a first aspect of the present invention, there is provided a system configured to selectively block respiratory air flow to a lung, comprising: a catheter having a proximal end and a distal end and a central lumen extending from the proximal end to the distal end, wherein the distal end of the catheter is closed, wherein the distal end of the catheter comprises a window configured to facilitate visualization beyond the window, wherein the catheter comprises an inflatable balloon positioned along a distal portion of the catheter, wherein the catheter comprises an inflation channel within a wall of the catheter surrounding the central lumen configured to facilitate inflation and deflation of the inflatable balloon; and a retention assembly configured to exert a force on a visualization device inserted within the central lumen of the catheter to cause a distal end of the visualization device to be pressed against the window at the distal end of the catheter. An endobronchial blocker is disclosed having an outer diameter of approximately 3 mm and having its own reversibly-coupled visualization system. The reversibly-coupled visualization system may advantageously allow the blocker to: i) be placed under direct vision without obstruction of a single-lumen endotracheal tube, ii) confirm appropriate expansion and seating within the chosen main stem bronchus, and/or iii) when pulled back proximal of a balloon of the blocker, can be left in place and used in real-time fashion to monitor the position of the blocker throughout the entire surgical procedure. Optionally, the visualization system is not an integral part of the endotracheal tube and can be moved separately from the endotracheal tube itself.

The endobronchial blocker described herein may be designed to be used with the visualization and/or cleaning devices and systems described in one or more of the following patent applications : WIPO Publ. No. WO 2013/063520, published on May 2, 2013 and U.S. Provisional Application No. 61/733,371, filed December 4, 2012. For example, the embodiments of endobronchial blockers described herein may be used in conjunction with the adapters or coupling members (e.g., adapters or coupling members 121, 2400, 2400', 2421, 2422, 2440, 2500, 2555) described in WIPO Publ. No. WO 2013/063520.

A system configured to selectively block respiratory air flow to a lung is provided. The system comprises a catheter having a proximal end and a distal end and a central lumen extending from the proximal end to the distal end. Optionally, the proximal end of the elongate member is open. The distal end of the elongate member is closed or sealed off to outside air. The distal end of the catheter comprises a window configured to facilitate visualization beyond the window. The catheter comprises an inflatable balloon positioned along a distal portion of the catheter. The catheter comprises an inflation channel within a wall of the catheter surrounding the central lumen configured to facilitate inflation and deflation of the balloon. The system comprises a retention assembly configured to exert a force on a visualization device (e.g., scope) inserted within the central lumen of the catheter to cause a distal end of the visualization device to be pressed against, the window at the distal end of the catheter. Non-inflatable expandable members may be used

Optionally, the catheter comprises a sheath along at least a portion of its length. At least a portion of the sheath of the catheter (e.g., a distal-most portion, such as the distal 1-6 cm, 0.5 cm - 1 cm, 1 cm - 4 cm, 1.5 cm - 5 cm, 2 cm - 8 cm, 3 cm - 6 cm, 4 cm - 10 cm) may be substantially transparent to allow for visualization outside of the wall of the catheter. In one embodiment, the entire sheath is at least substantially optically transparent or clear.

In some embodiments, the system comprises a balloon inflation control member at a proximal end of the catheter (e.g., to control inflation of a balloon disposed on the catheter). In one embodiment, the catheter comprises a second channel (e.g., auxiliary channel) within the wall of the catheter surrounding the central lumen. The second channel may comprise a distal opening or exit distal to the inflatable member (e.g., balloon) and proximal to the window or closed distal tip of the catheter to facilitate delivery of air and/or fluids to an airway of a patient through the second channel.

In some embodiments, the system comprises a visualization device (e.g., visualization scope) configured to be inserted within the central lumen of the catheter. In one embodiment, the system comprises a multi-port connector with two, three, four or more ports configured to be coupled to a proximal end of an endotracheal tube or other body-inserted tube. The catheter may be configured to be inserted within a port of the multi-port connector, through the endotracheal tube, and advanced to a location within a bronchus of a lung. In one embodiment, the system comprises a compression member (e.g. compression cap) that is configured to be coupled to the port of the multi-port connector that the visualization device is inserted within to provide compression of the visualization device within the catheter. In one embodiment, the compression member comprises a flexible diaphragm, gasket, O-ring and/or other seal member configured to receive the catheter. The diaphragm or other seal member may be configured to seal or otherwise close down around and conform to an outer diameter of the catheter. The compression member may be configured to exert a compressive force on the catheter to inhibit axial or rotational movement of the catheter once the catheter is in a desired position. The retention assembly may comprise a retention member configured to engage with or couple to a corresponding member on the visualization device (e.g., a notch, slot, groove, recess, protrusion, ring, detent, loop, adhesive member) and an elastomeric sleeve configured to stretch to facilitate engagement with the corresponding member and exert a returning force as a result of the tendency to return to a relaxed, non-stretched state.

Also disclosed is, a method for selectively blocking respiratory air flow through an endotracheal tube to one of a patient's lungs is provided. Optionally, the method comprises providing an endobronchial blocker such as the endobronchial blockers described herein.

The method may comprise coupling an endotracheal tube adapter having at least two inlet ports to an endotracheal tube within an intubated patient and inserting a visualization device within the central lumen of the endobronchial blocker. Optionally, the method comprises advancing a distal end of the visualization device to the distal end of the endobronchial blocker.

Optionally, the method comprises inserting the endobronchial blocker within a first inlet port of the two inlet ports and causing the retention assembly to exert the force on the visualization device by coupling a retention member of the retention assembly to the visualization device. Optionally, the method comprises advancing the distal end of the endobronchial blocker within one of the lungs of the patient. The method may comprise confirming the positioning of the distal end of the endobronchial blocker using the visualization device. Optionally, the method comprises uncoupling the visualization device from the retention assembly and withdrawing the visualization device past a proximal end of the inflatable balloon of the endobronchial blocker. Optionally, the method comprises inflating the inflatable balloon to occlude the lung or portion thereof and confirming proper inflation and positioning of the inflatable balloon using the visualization device.

Optionally, the method comprises coupling a ventilator to a second inlet port of the two inlet ports. The method may comprise recording an image of the position of the inflatable balloon within a bronchus. Optionally, the method comprises aspirating the airway beyond the inflatable balloon through a second channel within the wall of the catheter and/or insufflating the airway beyond the inflatable balloon through a second channel within the wall of the catheter.

Optionally, the method comprises inserting a suction catheter through a third inlet port of the endotracheal tube adapter. The method comprises inserting a fiberoptic bronchoscope through a third inlet port of the endotracheal tube adapter. Optionally, the method is performed without obstruction of the endotracheal tube or other body-inserted tube. Optionally, , the method comprises coupling a compression member to the catheter configured to exert a compressive force on the catheter to inhibit axial or rotational movement of the catheter once the catheter is in a desired position. Certain steps may be performed in a different order and/or may be optional.

For purposes of summarizing the disclosure, certain aspects, advantages and novel features of embodiments of the inventions have been described herein. It is to be understood that not necessarily all such advantages can be achieved in accordance with any particular embodiment of the inventions disclosed herein. Thus, the embodiments disclosed herein can be embodied or carried out in a manner that achieves or optimizes one advantage or group of advantages as taught herein without necessarily achieving other advantages as can be taught or suggested herein.

The disclosed methods summarized above and set forth in further detail below describe certain actions taken by a practitioner; however, it should be understood that they can also include the instruction of those actions by another party. For example, actions such as "inflating a balloon" include "instructing the inflating of a balloon." Further aspects of embodiments of the invention will be discussed in the following portions of the specification. With respect to the drawings, elements from one figure may be combined with elements from the other figures.

### BRIEF DESCRIPTION OF THE DRAWINGS

Several embodiments of the inventions disclosed herein will be more fully understood by reference to the following drawings which are for illustrative purposes only:
Figure 1A illustrates an embodiment of a tri-port connector that is advantageously designed to be used in conjunction with embodiments of the endobronchial blocker described herein.
Figure 1B schematically illustrates the tri-port connector of Figure 1A with the endobronchial blocker inserted through one of the ports of the tri-port connector.
Figures 2A and 2B schematically illustrate various views of an embodiment of a compression mechanism of the endobronchial blocker.
Figure 2C illustrates a side view of an alternative embodiment of a compression mechanism of the endobronchial blocker.
Figure 3 illustrates an embodiment of a retention mechanism of the endobronchial blocker.
Figures 4A and 4B schematically illustrate an embodiment of a distal end of the endobronchial blocker.
Figure 5 illustrates an embodiment of a proximal end of the endobronchial blocker.
Figure 6 illustrates an embodiment of a ventilation port of the tri-port connector of Figures 1A and 1B.
Figures 7 and 7A illustrate a configuration of caps covering a main port of the tri-port connector of Figures 1A and 1B.
Figures 8 and 8A illustrate a configuration of caps for the port of the tri-port connector of Figures 1A and 1B into which the endobronchial blocker is inserted.
Figures 9A and 9B illustrate an embodiment of the entire endobronchial blocker 120 in two different states of use.

### DETAILED DESCRIPTION

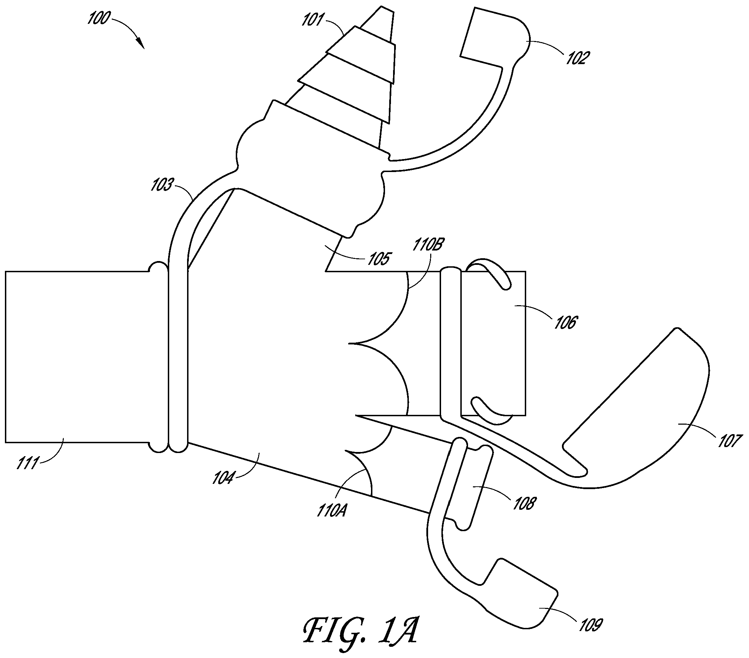

Figure 1A schematically depicts an embodiment of a tri-port connector 100 that is advantageously designed to work in conjunction with embodiments of the endobronchial blockers described herein. In accordance with some embodiments, the tri-port connector 100 comprises one or more features of the adapters, connectors or coupling members (e.g., adapters or coupling members 121, 2400, 2400', 2421, 2422, 2440, 2500, 2555), such as, e.g., those described in WIPO Publ. No. WO 2013/063520 Optionally , the tri-port connector 100 includes three ports and a universal endotracheal tube coupling member; however, the connector 100 may include fewer ports (e.g., two ports) or more ports (e.g., four ports, five ports, greater than five ports, etc.). The first port can include an oxygen port adapter 101 having an optional cap 102 and a tether 103 to a main manifold 104 of the tri-port connector 100. The first port of the tri-port connector 100 may also include a ventilator connection site 105 that becomes available when the oxygen port adapter 101 is removed.

The second port of the tri-port connector 100 is a main manifold port 106, which may also optionally include a cap 107. The main manifold port 106 can be configured to receive an intubation and/or cleaning system having visualization or imaging capabilities or functions (such as, for example, components of the visualization devices 120 or visualization device assembly 2521 or cleaning systems described in WIPO Publ. No. WO 2013/063520) and/or a cap-insert configured to allow suctioning, fiberoptic bronchoscopy or endoscopy, or other instrumentation of the tracheobronchial tree or other anatomical features during an operation or procedure.

In some embodiments, the third port of the tri-port connector 101 comprises a manifold port 108 through which a stylet (e.g., malleable stylet), obturator or other device can be placed (for example, at the time of intubation). The manifold port 108 can optionally include a cap 109 or similar seal member. In some embodiments, the tri-port connector 100 further includes a universal connection port 111 that connects the tri-port connector 100 to the proximal portion of standard endotracheal tubes or other medical tubes (e.g., tracheostomy tubes). As shown, the tri-port connector 100 may include one-way valves 110 (e.g., "duckbill" valves or flap valves), diaphragms, seals or other flow (e.g., backflow) prevention members that maintain positive pressure within the main manifold 104 (and ventilator circuit) when no devices are in place through the valves 110.

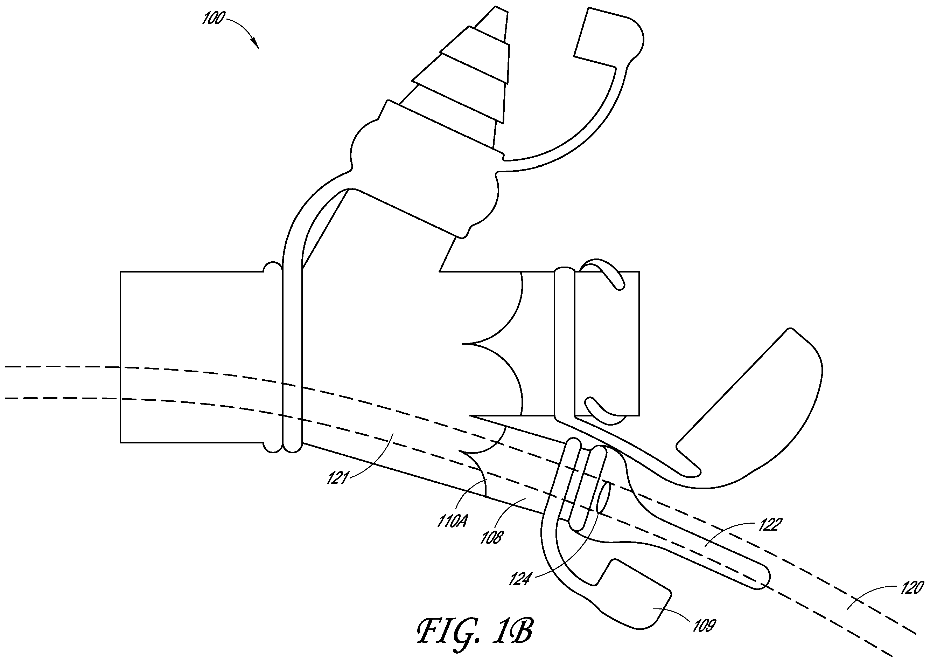

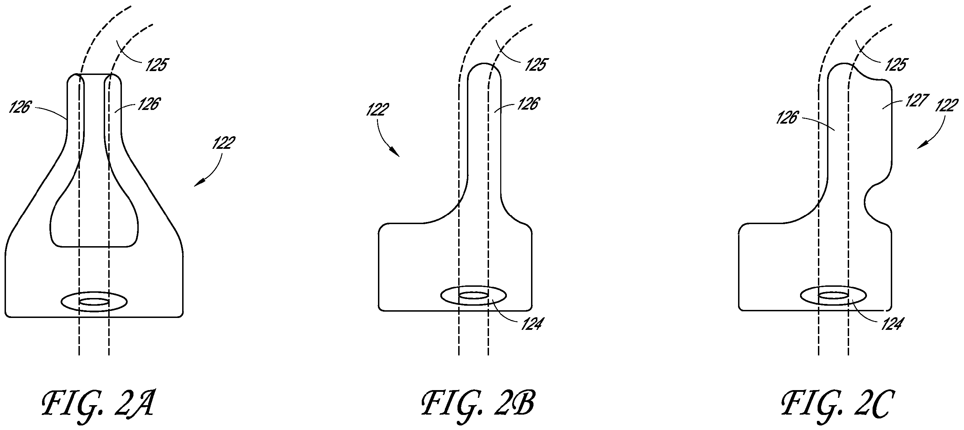

Figure 1B schematically illustrates the tri-port connector 100 with a portion of an embodiment of an endobronchial blocker 120 inserted through the manifold port 108. In some embodiments, the endobronchial blocker 120 is inserted through the manifold port 108 after removal of a malleable stylet or obturator (e.g., used during intubation). The one way valve 110A within the manifold port 108 is displaced by a catheter portion 121 of the endobronchial blocker 120 when inserted. Figure 1B also illustrates an embodiment of a compression cap 122 that is configured to be coupled to the manifold port 108. The compression cap 122 is configured to provide a compression site on a portion (e.g., visualization device and sheath assembly) of the blocker 120 so as to maintain the position of the blocker 120 (e.g., prevent or restrict longitudinal movement), as well as to maintain compression of a visualization device (e.g., fiber optic camera scope) within the blocker 120 so that the visualization device does not rotate within a surrounding sheath of the blocker 120. The compression features of the compression cap 122 are illustrated and described in more detail in connection with Figures 2A-2C. In some embodiments, the compression cap 122 comprises a soft and/or flexible diaphragm 124, valve or other seal member through which the catheter portion 121 of the blocker 120 can be placed. In one embodiment, the diaphragm 124 may advantageously prevent, or reduce the likelihood of, loss of positive pressure from the manifold 104 (and ventilatory circuit) while the endobronchial blocker 120 is in place.

Figures 2A and 2B illustrate front and side views, respectively, of a version of the compression cap 122 of Figure 1B. The compression cap 122 may be configured to be coupled to an endobronchial insertion port (e.g., manifold port 108) of a multi-port connector (e.g., tri-port connector 100). In some embodiments, the compression cap 122 is configured to receive, engage with and/or couple to an elastomeric and/or compressible sheath 125, inside of which a visualization device can be inserted (not shown).

Optionally, the elastomeric and/or compressible sheath 125 comprises a portion of the catheter portion 121 of the endobronchial blocker 120. The visualization device can comprise a fiber optic camera scope or any other type of scope (e.g., bronchoscope, endoscope) or visualization/imaging device.

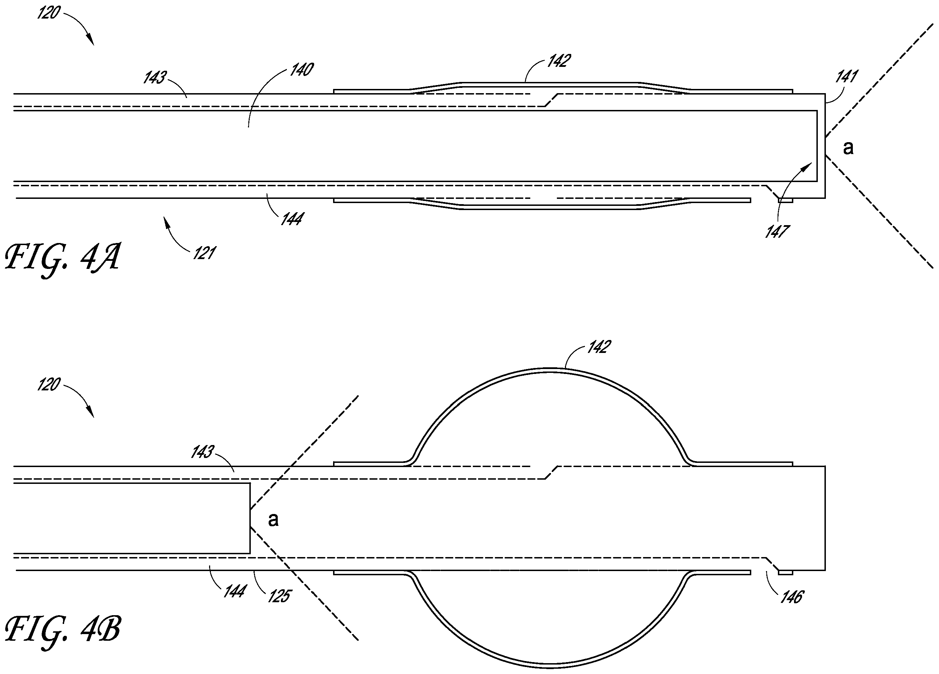

The visualization device may comprise a charge-coupled device (CCD) camera device, chip-on-stick CMOS imaging device, LED imaging, or ultrasound imaging device. Figures 2A and 2B further illustrate an embodiment of the flexible diaphragm 124 through which the endobronchial blocker 120 can be placed. The depicted compression cap 122 includes a compression channel formed between two compression members 126 into which the endobronchial blocker elastomeric sheath 125 is "snapped" or otherwise secured. Optionally, the compression members 126 provide compression of the sheath 125 against the visualization device (e.g., scope) within the sheath 125 and accomplish at least two functions. First, the compression members 126 can prevent migration of the distal end of the endobronchial blocker 120, which includes a lumen-occluding balloon (as shown in Figures 4A and 4B). Second, the compression members 126 can provide compression on the visualization device itself to prevent rotation of a camera or other imaging device at a distal end of the visualization device, which could result in undesirable rotation of the visual image as it is being monitored or acquired. The sheath 125 can comprise extruded silicone, urethane, TPE, latex, and/or other elastomeric or polymeric materials.

Figure 2C illustrates a side view of an alternative compression cap or mechanism 122 (e.g., a "hair clip"). As shown, the compression cap 122 of Figure 2C includes two "wings" 127 that when compressed toward each other open up the channel between the two compression members 126 such that the sheath 125 containing the visualization device (e.g., scope) can be laid or otherwise positioned within the compression channel without requiring significant pressure to "snap" it into place. Optionally, once the sheath 125 is positioned between the two compression members 126, the compressed "wings" 127 are released, thereby causing the compression members 126 to once again return to their resting position and apply compression to the sheath 125 surrounding the visualization device. The sheath 125 may be captured either by the natural recoil of the design or, alternatively, by a spring mechanism, which can be placed between the wings 127 so that when the wings 127 are released, the compression members 126 actively capture the sheath and visualization device assembly 123 (e.g., similar to a hair clip with opposing jaws or claws operated by a spring mechanism).

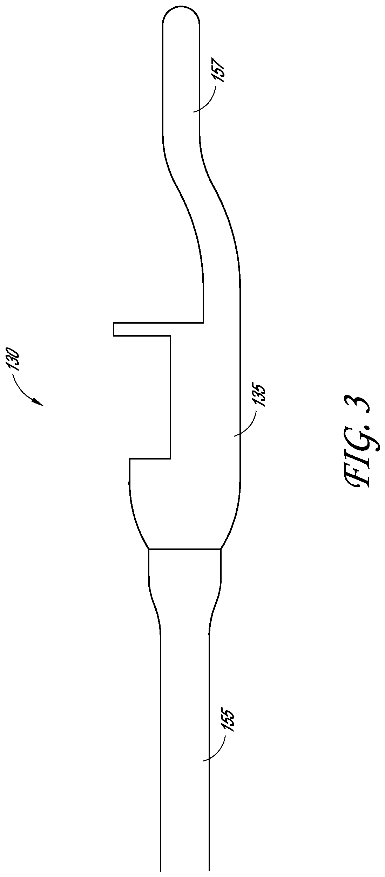

Figure 3 schematically depicts an embodiment of a visualization device retention assembly 130. The retention assembly 130 can include, for example, any of the features described in connection with the scope retention assemblies 123, 2725, 2825 described in WIPO Publ. No. WO 2013/063520. The retention assembly 130 can be used in conjunction with one or more of the endotracheal tube cleaning devices, visualization devices, and/or airway cleaning devices described in WIPO Publ. No. WO 2013/063520 Optionally, the retention assembly 130 includes an elastomeric sleeve 155, a retention member 135 and a compression member 157, which may operate and provide functions similar to the compression mechanism 122 identified and described in Figures 2A-2C above. A reverse bias or force can be exerted on a visualization device (e.g., visualization scope) by the retention member 135 (e.g., as a result of the stretchable elastomeric sleeve 155 wanting to return to its relaxed, non-stretched state) to advantageously press a distal visualization end of the visualization device against a window 141 at a distal end of the endobronchial blocker 120 (as shown in Figures 4A and 4B). Optionally, the visualization device comprises an engagement member that is configured to engage with the retention member 135 and is positioned at a location configured to allow the distal end of the visualization device to be held against, or in close proximity to, the window 141. Optionally, the retention assembly 130 can be reused several times (e.g., 100-1000) times while still maintaining its effectiveness. The materials for the sleeve 155 can include extruded silicone, urethane, TPE, latex, and/or other elastomeric or polymeric materials. In some embodiments, the maximum elongation of the sleeve 155 can range from approximately 150% to 750% (e.g., from about 250% to 500%, from about 150% to 600%, from about 300% to 550%, from about 350% to 600%, from about 400% to 450%, overlapping ranges thereof, or 425%)

Once a visualization device (e.g., scope) has been used to appropriately direct and place the endobronchial blocker 120 at a location within a tracheobronchial location (e.g., within a bronchus of a lung) or other anatomical location, the visualization device is uncoupled from the retention member 135 and pulled backward until appropriate visual identification and confirmation of balloon position of an occluding balloon of the endobronchial blocker (e.g., balloon 142 shown in Figures 4A and 4B) and tracheobronchial anatomy is obtained. At that point, the visualization device may then be "snapped" into position or otherwise coupled or positioned relative to the compression cap or mechanism 122 of Figures 2A and 2B or Figure 2C. Other designs and approaches of creating a static reverse force on the visualization device to improve the quality of visualization are possible without departing from the scope of the disclosure herein.

Figures 4A and 4B schematically illustrate a distal end of the endobronchial blocker 120. Figure 4A shows a distal end of a visualization scope 140 pressed up against a terminal optical window 141 of the catheter portion 121 of the blocker 120 and demonstrates the camera having at least a 90° angle of view, represented by angle *a*. A reverse bias or force can be exerted on the visualization scope 140 by the retention member 135 to advantageously press a viewing end 147 (e.g., lens end) of the visualization scope 140 against the window 141 at the distal end of the catheter portion 121 of the endobronchial blocker 120. Such a configuration may result in minimal or no air gap between the viewing end of the visualization scope 140 and the window 141. Optionally, the window thickness combined with the lens indentation is less than 0.254mm (e.g., 0.0254mm, 0.0508mm, 0.0762mm, 0.1016mm, 0.127mm, 0.1524mm, 0.1778mm, 0.2032mm, 0.2286mm, 0.254mm, 0.0254mm to 0.254mm, 0.125mm to 0.254mm, 0.2286mm to 0.254mm, 0.1524mm to 0.2286mm, or overlapping ranges thereof) (0.010 inches, e.g., 0.001 inches, 0.002 inches, 0.003 inches, 0.004 inches, 0.005 inches, 0.006 inches, 0.007 inches, 0.008 inches, 0.009 inches, 0.010 inches, 0.001 inches to 0.010 inches, 0.005 inches to 0.010 inches, 0.009 inches to 0.010 inches, 0.006 inches to 0.009 inches, or overlapping ranges thereof) in order to reduce glare and/or halo effects and otherwise improve the quality of visualization. This can be particularly helpful during a treatment procedure because glare may make it difficult to view one or more anatomical features. However, the clearance between a distal lens end of the visualization scope 140 and the window 141 of the endobronchial blocker 120 and/or the combined thickness of the window 141 and lens indentation can be different than disclosed herein. One or more antireflective coatings, layers or other features can be applied to the outside of the window 141 to further reduce glare. One or more elements to reduce condensation (e.g., anti-fogging) may be provided. For example, a heating element can be thermally coupled to the window 141. The heating element can heat up periodically, or as needed (e.g., as determined by a sensor), thereby warming the window 141 and preventing condensation or fog from forming on the window. Suction can be applied to the window 141 even in the absence of view-obstructing fluids because the application of suction would tend to cool the window 141 or remove vapor that might otherwise tend to condense on the window 141.

The window 141 can have a thickness of less than about 0.3048mm (for example, 0.0254mm, 0.0508mm, 0.0762mm, 0.1016mm, 0.127mm, 0.1524mm, 0.1778mm, 0.2032mm, 0.2286mm, 0.254mm, 0.274mm, 0.3048mm, etc.) (0.012 inches, for example, 0.001 inches, 0.002 inches, 0.003 inches, 0.004 inches, 0.005 inches, 0.006 inches, 0.007 inches, 0.008 inches, 0.009 inches, 0.010 inches, 0.01 1 inches, 0.012 inches, etc.). Optionally, the thickness of the window is about 0.127mm (0.005inches). In some embodiments, the thickness of the window does not exceed about 0.2032mm (0.008 inches). The window injection mold can be highly polished (e.g., with an SPE #1 finish and/or optical finish) or otherwise treated in order to ensure optical clarity of the molded parts. Optionally, the lens 147 of the visualization scope 140 is indented by a few hundreds of a mm (e.g., about 0.0254mm to about 0.1016mm, i.e. a few thousands of an inch, e.g. about 0.001 to about 0.004 inches) in order to prevent or reduce the likelihood of scratches and damage to the lens.

In Figure 4A, the endobronchial occluding balloon 142 is collapsed or deflated. The catheter portion 121 of the endobronchial blocker 120 includes a pilot balloon channel 143 and an additional channel 144 configured to allow some degree of continuous positive airway pressure (CPAP), aspiration, irrigation, and/or insufflation of the airway beyond the occluding balloon 142 (e.g., via opening 146). Optionally, the additional channel 144 comprises a ventilation channel.

Figure 4B schematically illustrates an embodiment of an occluding balloon 142 inflated and deployed within an appropriate bronchus with the distal end of the visualization scope 140 pulled back to a point such that the angle of view a shows the inflated balloon 142 and the surrounding tracheobronchial tree anatomy. Optionally, the view of the tracheobronchial tree anatomy is facilitated by optically clear components of the endobronchial blocker sheath 125 at this level. The angle of view a can be greater than 90° (e.g., 90°-100°, 95°-110°, 90°-120°, 100°-120°, or overlapping ranges thereof) or less than 90° (e.g., 80°-90°, 70°-85°, 60°-75°, or overlapping ranges thereof).

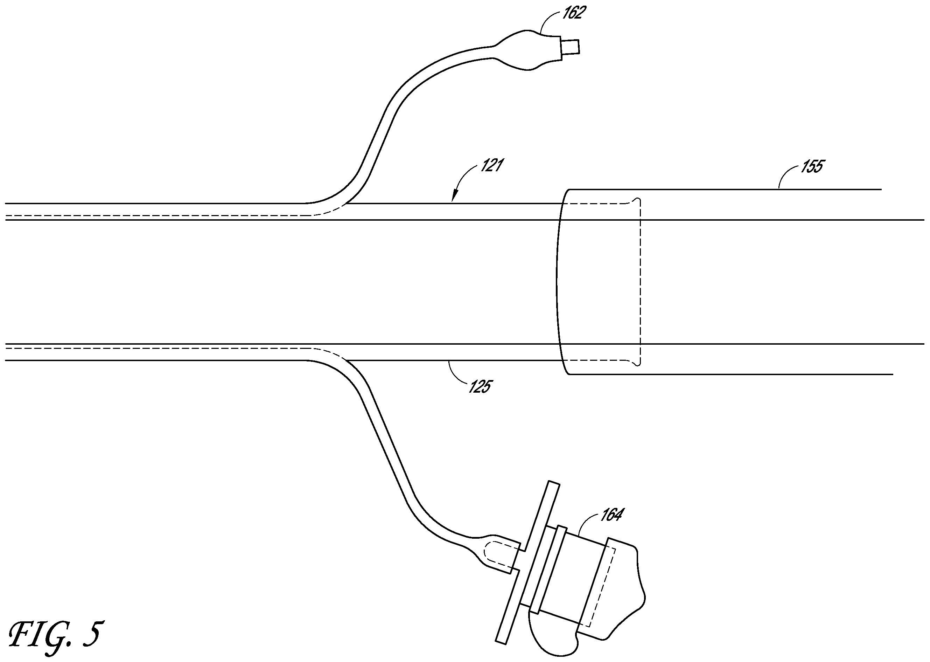

Figure 5 depicts a proximal portion of the endobronchial blocker 120. As shown, the endobronchial blocker 120 includes a balloon inflation control member 162. The inflation control member 162 can include a one-way valve and release for selective inflation and deflation of the occluding balloon 142 via the pilot balloon channel 143. Other seal members and/or or flow control mechanisms may also be used. Figure 5 also shows one illustrated embodiment of a ventilator connection member 164 configured for insufflation, aspiration, and/or or maintenance of some degree of CPAP with the balloon 142 deployed. The ventilator connection member 164 is in communication with the additional channel 144. Figure 5 further illustrates a distal end of the elastomeric sleeve 155 that provides at least a slight distal pressure of the visualization device distal tip (e.g., lens end of visualization scope) against the optical window 141 when used with the previously-described retention assembly 130. As previously described, the elastomeric sleeve 155, when compressed at some point along its length, can help prevent or reduce the likelihood of unwanted rotation of the visualization scope 140 within the endobronchial blocker sheath 125. Although the exits of the pilot balloon inflation control member 162 and the ventilator connection member 164 tubing from the catheter portion 121 are shown originating distal to the connection of the elastomeric sleeve 155 with the distal endobronchial blocker sheath 125, the exit point for these two structures can be located proximal to the connection of the elastomeric sleeve 155, as desired or required for a particular application or use.

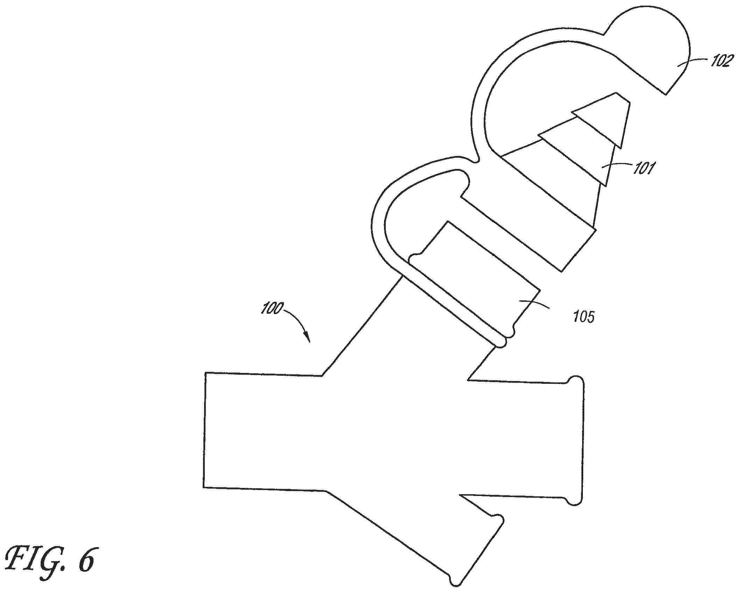

Figure 6 illustrates an exploded assembly view of one embodiment of the ventilation port of the tri-port connector 100. In the illustrated embodiment, the oxygen connection adapter 101 is a standard "Christmas tree"-style or similar connector for connection of oxygen tubing that is configured to flush the endotracheal tube to pool oxygen (e.g., 100% oxygen) in the posterior oropharynx of the patient during intubation, as well as to prevent splash back of either secretions or blood onto the optical window 141 of the blocker 120 during the intubation procedure. Figure 6 further illustrates the ventilator connection site 105 that is provided when the oxygen connection adapter 101 (e.g., "Christmas tree" connector) is removed and secured by the tether 103.

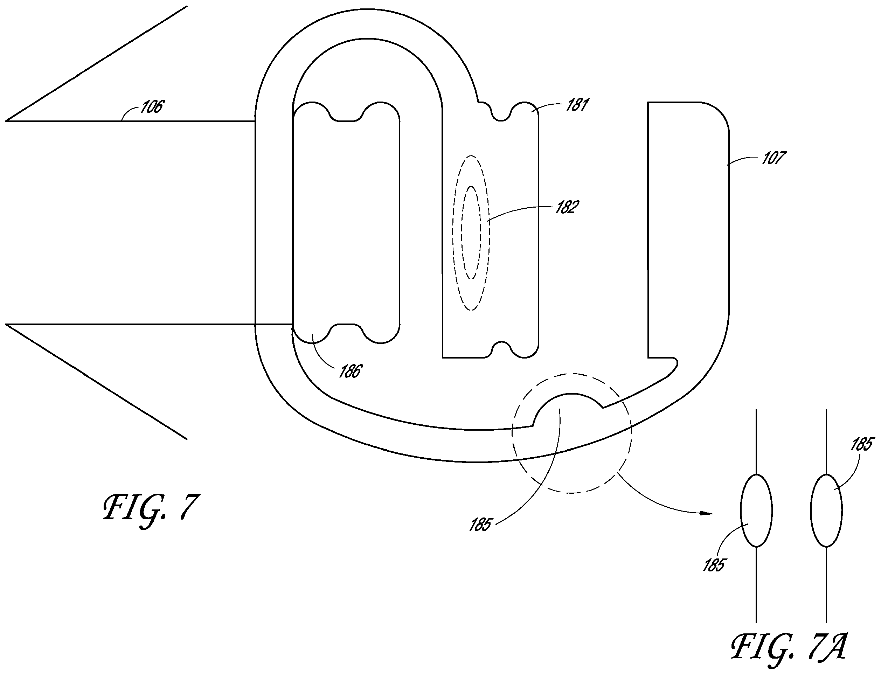

Figure 7 illustrates one embodiment of a configuration of caps or other seal members that can be used to cover the main manifold port 106 of the tri-port connector 100. As shown, the cap 107 can be a tethered solid cap that can close off the main manifold port 106 completely. In the illustrated embodiment, the cap 107 includes a second tethered cap member 181 with a central distensible diaphragm 182.

The diaphragm 182 can be configured to accommodate instruments such as a suction catheter or fiber-optic bronchoscope (e.g., instruments generally between 4 and 7 mm in diameter), and can seal around such instruments so that no leaks occur from the ventilatory circuit. Figure 7A illustrates a close-up view of a section of a tether of the cap 107 that includes two bumps, protrusions or "knobs" 185 that are sized to clamp onto a sleeve or sheath of a visualization device once it has been positioned for intubation with a camera at the distal end of the endotracheal tube. Clamping of the sleeve or sheath with these knobs 185 may advantageously prevent or reduce the likelihood of axial or radial movement of the camera within the endotracheal tube during the intubation procedure, similar to the compression cap 122 described herein. The main manifold port 106 can include a "bump" or protrusion 186 configured to prevent the tether from moving in an unwanted fashion towards the cap 107 once the knobs 185 have been clamped onto the visualization device sheath.

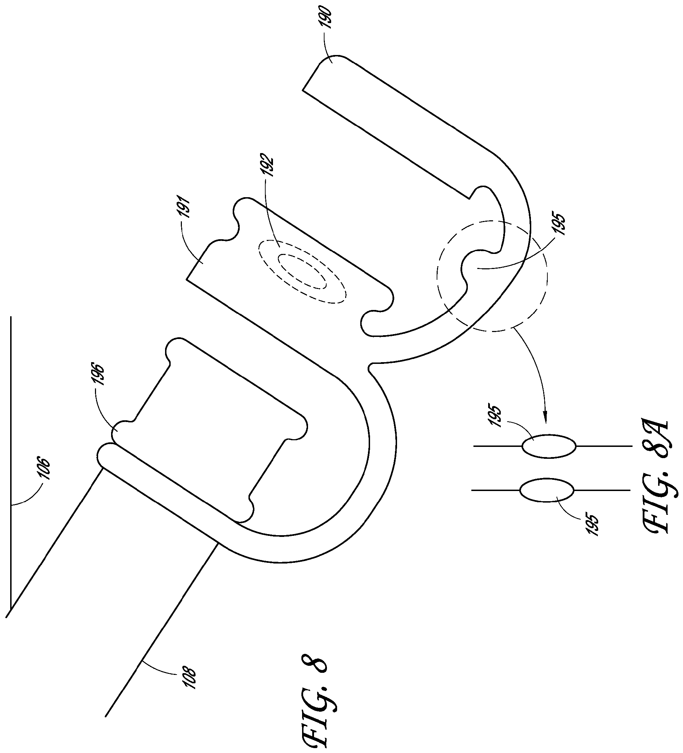

Figure 8 illustrates one embodiment of a configuration of a cap assembly that can be used for a stylet and blocker port (e.g., manifold port 108) of the tri-port connector 100. In the illustrated embodiment, the cap assembly includes a tethered solid cap 190 that can close off the stylet and blocker port 108 completely and a tethered cap 191 with a central distensible diaphragm 192 that can accommodate, for example, either an intubating stylet or the endobronchial blocker 120, or other device having an outer diameter of between about 1.5 mm and 5 mm. Figure 8A illustrates a close-up view of a section of a tether of the cap 190 that contains two bumps, protrusions, "knobs," or other features 195 that are sized to clamp onto the endobronchial blocker 120 or other device once it has been appropriately positioned and the balloon 142 inflated within an appropriate bronchus of the lungs. This clamping may advantageously help prevent, or reduce the likelihood of, migration of the blocker 120 during the procedure. The knobs 195 may provide functions similar to the compression cap 122 described herein. The manifold port 108 can include a "bump" or protrusion 196 that is configured to prevent the tether from interfering with functioning of the cap 191.

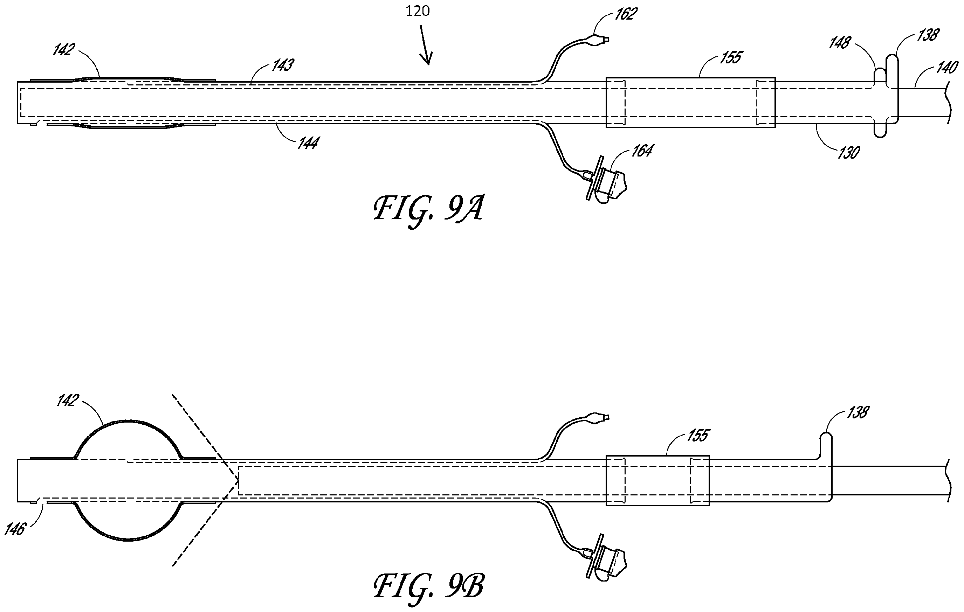

Figures 9A and 9B illustrate an embodiment of the entire endobronchial blocker 120 in two different states of use. Figure 9A illustrates a state of use with the visualization scope 140 inserted within the main lumen of the endobronchial blocker 120 and engaged with the scope retention assembly 130. As described above, the scope retention assembly 130 may include a retention member 138 that is configured to engage with a corresponding retention member 148 on the visualization scope 140. As shown in Figure 9A, with the retention member 138 engaged with the retention member 148, the distal end of the visualization scope 140 is pressed against the window 141 at the distal end of the endobronchial blocker 120.

Figure 9B illustrates a state of use wherein the balloon 142 is inflated and the visualization scope 140 has been disengaged from the retention member 138 and withdrawn to a position proximal to the balloon 142 to facilitate visualization of the inflated balloon 142 through the transparent or optically clear wall of the catheter portion 121 of the endobronchial blocker 120, as described in more detail above. In some embodiments, the entire catheter portion 121 of the endobronchial blocker 120 is transparent or optically clear other than the elastomeric sleeve 155. Optionally, at least a portion of the catheter portion 121 proximal to the balloon 142 is transparent or optically clear sufficient to facilitate visualization of the balloon 142.

The endobronchial blocker 120 can be inserted within the tri-port connector 100 after performing a routine intubation. The intubation may be facilitated through the use of a visualization or imaging system, such as one or more of the visualization systems described in WIPO Publication Number WO 2013/063520, which may be used to confirm proper positioning of placement of a distal end of an endotracheal tube during intubation of a patient using direct visualization. A patient may be anesthetized and intubated with a single-lumen endotracheal tube or other body-inserted medical tube according to clinician preference. The endotracheal tube may be secured to the patient. Optionally, the lumen of the endotracheal tube is from 5 mm to 9 mm.

Optionally, the tri-port connector 100 is inserted between the endotracheal tube and a ventilator, with the ventilator connected to a ventilator port of the tri-port connector 100 and the endotracheal tube connected to the universal connection port 111. Optionally, the endobrochial blocker 120 is inserted through the stylet port (e.g., manifold port 108) of the tri-port connector 100 and advanced distally through the endotracheal tube. Optionally, with the assistance of visualization provided by the camera at the tip of the endobronchial blocker 120 behind an optically clear window 141, the images of which are displayed on a monitor, the distal end of the endobronchial blocker 120 is directed into the chosen bronchus for occlusion. The monitor or a storage device coupled to the monitor can store video or still images obtained and/or transmit the images to a remote location. Steering may be provided by slight angulation near the tip of the endobronchial blocker 120. Visualization on the compatible monitor can also allow for confirmation of placement of the distal tip of the endotracheal tube. visualization scope 140 may then be withdrawn proximally to the origin of the balloon 142 and the balloon 142 may be inflated under direct vision to be certain that it inflates appropriately and in the correct position. Tracheobronchial landmarks, such as the carina and non-occluded bronchus or bronchi, can be easily viewed through a transparent or substantially transparent catheter portion 121 proximal to the origin of the occluding balloon 142.

Continuously or intermittently (e.g., at certain times during the procedure), the position of the balloon 142 can be easily confirmed and adjusted as desired or necessary, as the visualization scope 140 remains in place in the catheter portion 121 of the endobronchial blocker 120 throughout the duration of the operative procedure. Once the procedure or treatment has been completed, the balloon 142 is deflated and the endobronchial blocker 120 may be removed.

The materials used for the various components of the connectors and endobronchial blockers described herein can advantageously comprise one or more biocompatible materials. Such materials can be rigid or semi-rigid and/or flexible, as desired or required for a particular application or use. The materials used can include, but are not limited to, polyether ether ketone (PEEK), Nylon 6/6, polyethylene, polypropylene, polyethylene terephthalate (PET), glycol-modified PET, polyvinyl chloride (PVC), thermoplastic elastomers (TPEs) such as PEBAX TPEs, other natural or synthetic polymers (e.g., KRATON polymers), silicone, natural rubber, latex, polycarbonate, K resin, acrylonitrile butadiene styrene (ABS), styrenes and/or other thermoplastic elastomers or polymers. The caps disclosed herein may be tethered or non-tethered.
The removable caps may be configured to be coupled via threaded coupling, snap-fit coupling, friction-fit coupling and/or any other type of connection device or method. The diaphragms or other seal member may be configured to seal or otherwise close down around and conform to an outer diameter of the devices inserted therethrough.

While the invention is susceptible to various modifications, and alternative forms, specific examples thereof have been shown in the drawings and are herein described in detail. It should be understood, however, that the invention is not to be limited to the particular forms disclosed, but to the contrary, the invention is to cover all modifications, equivalents, and alternatives falling within the scope of the appended claims. Any methods disclosed herein need not be performed in the order recited. The methods disclosed herein include certain actions taken by a practitioner; however, they can also include any third-party instruction of those actions, either expressly or by implication. For example, actions such as "inflating a balloon" include "instructing the inflating of a balloon."

Various embodiments of the invention have been presented in a range format. It should be understood that the description in range format is merely for convenience and brevity and should not be construed as an inflexible limitation on the scope of the invention. The ranges disclosed herein encompass any and all overlap, sub-ranges, and combinations thereof, as well as individual numerical values within that range. For example, description of a range such as from 70 to 115 degrees should be considered to have specifically disclosed subranges such as from 70 to 80 degrees, from 70 to 100 degrees, from 70 to 110 degrees, from 80 to 100 degrees etc., as well as individual numbers within that range, for example, 70, 80, 90, 95, 100, 70.5, 90.5 and any whole and partial increments therebetween. Language such as "up to," "at least," "greater than," "less than," "between," and the like includes the number recited. Numbers preceded by a term such as "about" or "approximately" include the recited numbers. For example, "about 10%" includes "10%." For example, the terms "approximately", "about", and "substantially" as used herein represent an amount close to the stated amount that still performs a desired function or achieves a desired result.

## Claims

1. A system configured to selectively block respiratory air flow to a lung, comprising:
a catheter (121) having a proximal end and a distal end and a central lumen extending from the proximal end to the distal end,
wherein the distal end of the catheter is closed,
wherein the distal end of the catheter comprises a window (141) configured to facilitate visualization beyond the window (141),
wherein the catheter comprises an inflatable balloon (142) positioned along a distal portion of the catheter,
wherein the catheter comprises an inflation channel (143) within a wall of the catheter surrounding the central lumen configured to facilitate inflation and deflation of the inflatable balloon (142); and
a retention assembly (130) configured to exert a force on a visualization device (140) inserted within the central lumen of the catheter to cause a distal end of the visualization device (140) to be pressed against the window (141) at the distal end of the catheter.

2. The system of Claim 1, wherein at least a portion of the catheter is at least substantially transparent to allow for visualization outside of the wall of the catheter.

3. The system of Claim 1, further comprising a balloon inflation control member (162) at a proximal end of the catheter.

4. The system of any of Claims 1-3, further comprising a second channel (144) within the wall of the catheter surrounding the central lumen, wherein the second channel comprises a distal opening (146) adjacent the distal end of the catheter to facilitate delivery of air or fluids to an airway of a patient through the second channel.

5. The system of any of Claims 1-3, further comprising a visualization scope (140) configured to be inserted within the central lumen of the catheter.

6. The system of any of Claims 1-3, further comprising a tri-port connector (100) configured to be coupled to a proximal end of an endotracheal tube.

7. The system of Claim 6, wherein the catheter is configured to be inserted within a port of the tri-port connector (100).

8. The system of Claim 7, further comprising a compression member (126) that is configured to be coupled to the port of the tri-port connector (100).

9. The system of Claim 8, wherein the compression member comprises a flexible diaphragm (124) configured to receive the catheter.

10. The system of Claim 8 or 9, wherein the compression member is configured to exert a compressive force on the catheter sufficient to inhibit axial or rotational movement of the catheter once the catheter is in a desired position.

11. The system of any of Claims 1-3, wherein the retention assembly (130) comprises a visualization device retention member (135) and an elastomeric sleeve (155).

12. The system of Claim 1, wherein an entire length of the catheter is comprised of optically clear material to facilitate visualization outside of the catheter.

13. The system of Claim 4, wherein the distal opening is distal of the inflatable balloon (142).

14. The system of any of Claims 6 to 9, further comprising an oxygen connection adapter (101) adapted to couple to a ventilator connection site of the tri-port connector (100).

15. The system of Claim 14, wherein the oxygen connection adapter (101) comprises a cap (102) and a tether (103).

## Patentansprüche

1. System, das dazu konfiguriert ist, den Atemluftstrom zu einer Lunge selektiv zu blockieren, umfassend:
einen Katheter (121) mit einem proximalen Ende und einem distalen Ende und einem zentralen Lumen, das sich von dem proximalen Ende zu dem distalen Ende erstreckt,
wobei das distale Ende des Katheters geschlossen ist,
wobei das distale Ende des Katheters ein Fenster (141) umfasst, das dazu konfiguriert ist, die Visualisierung hinter dem Fenster (141) zu ermöglichen,
wobei der Katheter einen aufblasbaren Ballon (142) umfasst, der entlang einem distalen Abschnitt des Katheters positioniert ist,
wobei der Katheter einen Aufblaskanal (143) in einer das zentrale Lumen umgebenden Wand des Katheters umfasst, der dazu konfiguriert ist, das Aufblasen und Entleeren des aufblasbaren Ballons (142) zu ermöglichen; und
eine Halteanordnung (130), die dazu konfiguriert ist, eine Kraft auf eine in das zentrale Lumen des Katheters eingeführte Visualisierungsvorrichtung (140) auszuüben, um zu bewirken, dass ein distales Ende der Visualisierungsvorrichtung (140) an das Fenster (141) an dem distalen Ende des Katheters gedrückt wird.

2. System nach Anspruch 1, wobei mindestens ein Abschnitt des Katheters mindestens im Wesentlichen durchsichtig ist, um die Visualisierung außerhalb der Wand des Katheters zuzulassen.

3. System nach Anspruch 1, weiter umfassend ein Ballonaufblassteuerelement (162) an einem proximalen Ende des Katheters.

4. System nach einem der Ansprüche 1-3, weiter umfassend einen zweiten Kanal (144) in der das zentrale Lumen umgebenden Wand des Katheters, wobei der zweite Kanal eine dem distalen Ende des Katheters benachbarte distale Öffnung (146) umfasst, um das Zuführen von Luft oder Fluiden zu einem Luftweg eines Patienten durch den zweiten Kanal zu ermöglichen.

5. System nach einem der Ansprüche 1-3, weiter umfassend ein Visualisierungs-Endoskop (140), das dazu konfiguriert ist, in das zentrale Lumen des Katheters eingeführt zu werden.

6. System nach einem der Ansprüche 1-3, weiter umfassend einen Drei-Port-Verbinder (100), der dazu konfiguriert ist, an ein proximales Ende eines Endotrachealtubus gekoppelt zu werden.

7. System nach Anspruch 6, wobei der Katheter dazu konfiguriert ist, in einen Port des Drei-Port-Verbinders (100) eingeführt zu werden.

8. System nach Anspruch 7, weiter umfassend ein Druckelement (126), das dazu konfiguriert ist, an den Port des Drei-Port-Verbinders (100) gekoppelt zu werden.

9. System nach Anspruch 8, wobei das Druckelement eine biegsame Membran (124) umfasst, die dazu konfiguriert ist, den Katheter aufzunehmen.

10. System nach Anspruch 8 oder 9, wobei das Druckelement dazu konfiguriert ist, eine Druckkraft auf den Katheter auszuüben, die ausreicht, um die Axial- oder Drehbewegung des Katheters zu verhindern, sobald sich der Katheter in einer gewünschten Position befindet.

11. System nach einem der Ansprüche 1-3, wobei die Halteanordnung (130) ein Visualisierungsvorrichtungs-Halteelement (135) und eine Elastomerhülle (155) umfasst.

12. System nach Anspruch 1, wobei eine gesamte Länge des Katheters aus optisch durchsichtigem Material besteht, um die Visualisierung außerhalb des Katheters zu ermöglichen.

13. System nach Anspruch 4, wobei die distale Öffnung distal des aufblasbaren Ballons (142) liegt.

14. System nach einem der Ansprüche 6 bis 9, weiter umfassend einen Sauerstoffverbindungsadapter (101), der zum Koppeln an eine Beatmungsgerät-Verbindungsstelle des Drei-Port-Verbinders (100) angepasst ist.

15. System nach Anspruch 14, wobei der Sauerstoffverbindungsadapter (101) eine Kappe (102) und eine Leine (103) umfasst.

## Revendications

1. Système configuré pour bloquer sélectivement le flux d'air respiratoire à un poumon, comprenant :
un cathéter (121) ayant une extrémité proximale et une extrémité distale et une lumière centrale s'étendant de l'extrémité proximale à l'extrémité distale,
dans lequel l'extrémité distale du cathéter est fermée,
dans lequel l'extrémité distale du cathéter comprend une fenêtre (141) configurée pour faciliter la visualisation au-delà de la fenêtre (141),
dans lequel le cathéter comprend un ballonnet gonflable (142) positionné le long d'une partie distale du cathéter,
dans lequel le cathéter comprend un canal de gonflage (143) au sein d'une paroi du cathéter entourant la lumière centrale configuré pour faciliter le gonflage et le dégonflage du ballon gonflable (142) ; et
un assemblage de retenue (130) configuré pour exercer une force sur un dispositif de visualisation (140) inséré au sein de la lumière centrale du cathéter pour amener une extrémité distale du dispositif de visualisation (140) à être pressée contre la fenêtre (141) au niveau de l'extrémité distale du cathéter.

2. Système selon la revendication 1, dans lequel au moins une partie du cathéter est au moins substantiellement transparente pour permettre la visualisation à l'extérieur de la paroi du cathéter.

3. Système selon la revendication 1, comprenant en outre un élément de commande de gonflage de ballonnet (162) au niveau d'une extrémité proximale du cathéter.

4. Système selon l'une quelconque des revendications 1 à 3, comprenant en outre un deuxième canal (144) au sein de la paroi du cathéter entourant la lumière centrale, dans lequel le deuxième canal comprend une ouverture distale (146) adjacente à l'extrémité distale du cathéter pour faciliter la distribution d'air ou de fluides à une voie respiratoire d'un patient à travers le deuxième canal.

5. Système selon l'une quelconque des revendications 1 à 3, comprenant en outre un endoscope de visualisation (140) configuré pour être inséré au sein de la lumière centrale du cathéter.

6. Système selon l'une quelconque des revendications 1 à 3, comprenant en outre un connecteur à trois ports (100) configuré pour être couplé à une extrémité proximale d'un tube endotrachéal.

7. Système selon la revendication 6, dans lequel le cathéter est configuré pour être inséré au sein d'un port du connecteur à trois ports (100).

8. Système selon la revendication 7, comprenant en outre un élément de compression (126) qui est configuré pour être couplé au port du connecteur à trois ports (100).

9. Système selon la revendication 8, dans lequel l'élément de compression comprend un diaphragme flexible (124) configuré pour recevoir le cathéter.

10. Système selon la revendication 8 ou 9, dans lequel l'élément de compression est configuré pour exercer une force de compression sur le cathéter suffisante pour inhiber le mouvement axial ou rotatif du cathéter une fois que le cathéter est dans une position souhaitée.

11. Système selon l'une quelconque des revendications 1 à 3, dans lequel l'assemblage de retenue (130) comprend un élément de retenue de dispositif de visualisation (135) et un manchon élastomère (155).

12. Système selon la revendication 1, dans lequel une longueur entière du cathéter est constituée d'un matériau translucide optiquement pour faciliter la visualisation à l'extérieur du cathéter.

13. Système selon la revendication 4, dans lequel l'ouverture distale est distale par rapport au ballonnet gonflable (142).

14. Système selon l'une quelconque des revendications 6 à 9, comprenant en outre un adaptateur de connexion d'oxygène (101) conçu pour être couplé à un site de connexion de ventilateur du connecteur à trois ports (100).

15. Système selon la revendication 14, dans lequel l'adaptateur de connexion d'oxygène (101) comprend un capuchon (102) et une attache (103).
